(19) Europäisches Patentamt
European Patent Office
Office européen des brevets

(11) EP 1 609 471 A1

(12) # EUROPEAN PATENT APPLICATION
published in accordance with Art. 158(3) EPC

(43) Date of publication:
**28.12.2005 Bulletin 2005/52**

(21) Application number: **04724790.3**

(22) Date of filing: **31.03.2004**

(51) Int Cl.[7]: **A61K 31/443**, A61P 11/00
// C07D405:06

(86) International application number:
**PCT/JP2004/004611**

(87) International publication number:
**WO 2004/087148 (14.10.2004 Gazette 2004/42)**

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **31.03.2003 JP 2003094504**

(71) Applicant: **KYOWA HAKKO KOGYO CO., LTD.**
**Chiyoda-ku, Tokyo 100-8185 (JP)**

(72) Inventors:
- **ABE, Yuzuru, 1008185 (JP)**
- **MIKI, Ichiro, 1008185 (JP)**

(74) Representative: **Casalonga, Axel**
**BUREAU D.A. CASALONGA - JOSSE**
**Paul-Heyse-Strasse 33**
**80336 München (DE)**

(54) **REMEDY AND/OR PREVENTIVE FOR LUNG DISEASES**

(57) The present invention provides a therapeutic and/or preventive agent for pulmonary disease that exhibits neutrophilic inflammation, such as, chronic obstructive pulmonary disease (COPD), pulmonary emphysema, chronic bronchitis, acute respiratory distress syndrome (ARDS), acute lung injury (ALI) and the like, which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof as an active ingredient.

EP 1 609 471 A1

## Description

Technical Field

The present invention relates to an agent for the treatment and/or prevention of pulmonary disease that exhibits neutrophilic inflammation.

Background Art

Chronic obstructive pulmonary disease (COPD), pulmonary emphysema, chronic bronchitis, acute respiratory distress syndrome (ARDS), acute lung injury (ALI) and the like are the pulmonary disease which are characterized by an onset of chronic neutrophilic inflammation [American Review of Respiratory Diseases (Am. Rev. Respir. Dis.), 1989, vol. 140, p. 1527; American Journal of Respiratory and Critical Care Medicine (Am. J. Respir. Crit. Care Med.), 1996, vol. 153, p. 530; and Current Opinion in Critical Care (Curr. Opin. Crit. Care), 2001, vol. 7, p. 1]. β-Stimulants, anticholinergic drugs, bronchodilators such as theophylline, and the like are used for pharmacotherapy of COPD, but they do not lead to drastic treatments for COPD [American Journal of Respiratory and Critical Care Medicine (Am. J. Respir. Crit. Care Med.), 2001, vol. 163, p. 1256]. In recent years, pharmacotherapy of COPD using phosphodiesterase (PDE) -IV inhibitors has been focused [Clinical and Experimental Allergy (Clin. Exp. Allergy), 1999, vol. 29, p. 99; and Lancet, 2001, vol. 358, p. 265].

On the other hand, ARDS and ALI are considered as inflammatory lesions due to injuries to, for example, pulmonary capillaries, pulmonary alveoli and the like. As the treatment of these diseases, causal treatment and symptomatic treatment such as the countermeasures to respiratory failure, and alternatively the administration of steroids are usually used [American Journal of Respiratory and Critical Care Medicine (Am. J. Respir. Crit. Care Med.), 1994, vol. 149, p. 818; and New England Journal of Medicine (N. Engl. J. Med.), 2000, vol. 342, p.1334].

Conventionally, it is known that 7-[2-(3,5-dichloro-4-pyridyl)-1-oxoethyl]-4-methoxy-spiro[1,3-benzodioxol-2,1'-cyclopentane] or a pharmaceutically acceptable salt thereof is used as a phosphodiesterase IV inhibitor (WO96/36624).

Disclosure of Invention

It is an object of the present invention to provide a therapeutic and/or preventive agent for pulmonary disease that exhibits neutrophilic inflammation, for example, COPD, pulmonary emphysema, chronic bronchitis, ARDS, ALI and the like, which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof as an active ingredient.

The present invention relates to the following (1) to (9).

(1) A therapeutic and/or preventive agent for pulmonary disease that exhibits neutrophilic inflammation, which comprises 7-[2-(3,5-dichloro-4-pyridyl)-1-oxoethyl]-4-methoxy-spiro[1,3-benzodioxol-2,1'-cyclopentane] represented by formula (I)

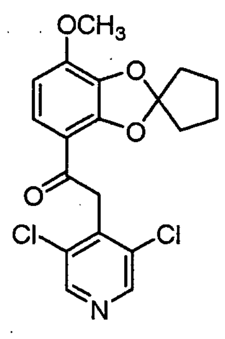

(I)

or a pharmaceutically acceptable salt thereof as an active ingredient.

(2) The therapeutic and/or preventive agent for pulmonary disease that exhibits neutrophilic inflammation according to (1), wherein the pulmonary disease that exhibits neutrophilic inflammation is a disease selected from the group consisting of COPD, pulmonary emphysema and chronic bronchitis.

(3) The therapeutic and/or preventive agent for pulmonary disease that exhibits neutrophilic inflammation according to (1), wherein the pulmonary disease that exhibits neutrophilic inflammation is ARDS or ALI.

(4) A method for treating and/or preventing pulmonary disease that exhibits neutrophilic inflammation, which comprises administering an effective amount of 7-[2-(3,5-dichloro-4-pyridyl)-1-oxoethyl]-4-methoxy-spiro[1,3-benzodioxol-2,1'-cyclopentane] represented by formula (I)

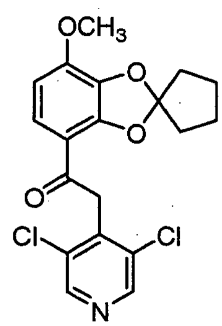

(I)

or a pharmaceutically acceptable salt thereof.

(5) The method for treating and/or preventing pulmonary disease according to (4), wherein the pulmonary disease that exhibits neutrophilic inflammation is a disease selected from the group consisting of COPD, pulmonary emphysema and chronic bronchitis.

(6) The method for treating and/or preventing pulmonary disease according to (4), wherein the pulmonary disease that exhibits neutrophilic inflammation is ARDS or ALI.

(7) Use of 7-[2-(3,5-dichloro-4-pyridyl)-1-oxoethyl]-4-methoxy-spiro[1,3-benzodioxol-2,1'-,cyclopentane] represented by formula (I)

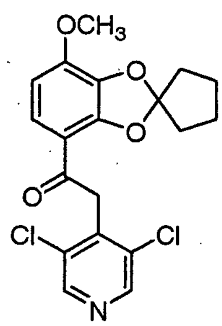

(I)

or a pharmaceutically acceptable salt thereof, for the manufacture of a therapeutic and/or preventive agent for pulmonary disease that exhibits neutrophilic inflammation.

(8) Use of 7-[2-(3,5-dichloro-4-pyridyl)-1-oxoethyl]-4-methoxy-spiro[1,3-benzodioxol-2,1'-cyclopentane] or a pharmaceutically acceptable salt thereof according to (7), wherein the pulmonary disease that exhibits neutrophilic inflammation is a disease selected from the group consisting of COPD, pulmonary emphysema and chronic bronchitis.

(9) Use of 7-[2-(3,5-dichloro-4-pyridyl)-1-oxoethyl]-4-methoxy-spiro[1,3-benzodioxol-2,1'-cyclopentane] or a pharmaceutically acceptable salt thereof according to (7), wherein the pulmonary disease that exhibits neutrophilic inflammation is ARDS or ALI.

Hereinafter, a compound represented by formula (I) is referred to as "Compound (I)".

Examples of the pharmaceutically acceptable salt of Compound (I) include acid addition salts, metal salts, ammonium salts, organic amine addition salts, amino acid addition salts and the like that are pharmaceutically acceptable.

Examples of the pharmaceutically acceptable acid addition salts of Compound (I) include inorganic acid salts

such as a hydrochloride, a sulfate, a nitrate and a phosphate; and organic acid salts such as an acetate, a maleate, a fumarate and a citrate. Examples of the pharmaceutically acceptable metal salt include alkali metal salts such as a sodium salt and a potassium salt; alkaline-earth metal salts such as a magnesium salt and a calcium salt; an aluminum salt; a zinc salt and the like. Examples of the pharmaceutically acceptable ammonium salts include an ammonium salt, a tetramethylammonium salt and the like. Examples of the pharmaceutically acceptable organic amine addition salts include addition salts of morpholine, piperidine or the like. Examples of the pharmaceutically acceptable amino acid addition salts include addition salts of glycine, phenylalanine, lysine, aspartic acid, glutamic acid or the like.

Next, a method for preparing Compound (I) will be described below.

Compound (I) can be prepared according to the method disclosed in WO96/36624.

Among Compound (I), stereoisomers such as tautomers may be existed, and including such isomers, all possible isomers and mixtures thereof can be used as the therapeutic and/or preventive agents for pulmonary diseases of the present invention.

To obtain a salt of Compound (I), when Compound (I) is obtained in the form of a salt, it may be purified as it is. When Compound (I) is obtained in the free form, Compound (I) may be dissolved or suspended in a suitable solvent, followed by addition of an acid or a base to form a salt. Then, the resulting salt may be isolated and purified.

Furthermore, Compound (I) and a pharmaceutically acceptable salt thereof may exist in the form of adducts with water or various solvents. These adducts can also be used as the therapeutic and/or preventive agents for pulmonary diseases of the present invention.

Pharmacological effects of Compound (I) will be described in detail based on the test examples.

TEST EXAMPLE 1: Inhibitory effect on increase of neutrophils in lipopolysaccharide (LPS)-induced pulmonary injury model

Physiological saline containing 25% Alevaire (registered trademark, Azwell Inc., Osaka) which is an inhalant for respiratory organs (administration solvent), or administration solvent dissolving 300 ng/mL of LPS (manufactured by Sigma-Aldrich, MO, USA) were administered intratracheally to 9-week-old male BALB/c mice (Charles River Japan, Kanagawa) in an amount of 0.1 mL respectively. Then bronchoalveolar lavages (BALs) were performed 6 hours after the intratracheal administration (These administration groups were referred to as a "solvent administration group" and an "LPS administration group", respectively). On the other hand, Compound (I) and LPS were suspended in an administration solvent to the concentration of 1 mg/mL and 300 ng/mL, respectively (The suspension is referred to as a "suspension for the administration of Compound (I)"). Then, 0.1 mL of the suspension for the administration was administered intratracheally. Bronchoalveolar lavage (BAL) was performed 6 hours after the intratracheal administration [Compound (I) administration group].

The recovered bronchoalveolar lavage fluids (BALFs) were centrifuged at 570×g for 10 minutes at 4°C, followed by removal of supernatants to obtain pellets. Each of the pellets was resuspended in 0.1 mL of physiological saline. The total numbers of leukocytes were counted with an automatic blood cell counter Celltac $\alpha$ (Nihon Kohden Corporation, Tokyo). After the counting, 0.2 mL of physiological saline was added to about 0.05 mL of each of remaining pellet suspensions, and then smeas were prepared using Cytospin 3 (Shandon, Pittsburgh, PA, USA). The smears were stained with Wright's stain (MICROX, Omron) using an automatic staining apparatus (Omron, Kyoto). Then, the numbers of cells were counted under a microscope (400x).

The number of cells was counted up to 300 in total while macrophages, neutrophils and lymphocytes were distinguished. Then, the ratio of each cell was calculated (equation 1). The number of neutrophils was calculated from the ratio of neutrophils and the total number of leukocytes (equation 2). The inhibition ratio of the increase in number of neutrophils by the administration of Compound (I) was calculated by equation 3. Cells in the BALFs of all individuals in this test were almost exclusively constituted of macrophages, neutrophils and lymphocytes. Eosinophils, basophils and other cells were hardly observed.

Table 1 shows the results on the number of neutrophils.

(equation 1) ratio of cells (%) = (number of Cells counted

under microscope/300) × 100

(equation 2) Number of neutrophils = (total number of

leukocytes × ratio of neutrophil)/100

(equation 3) Inhibition ratio of increase in number of neutrophils = 1 - {(number of neutrophils of Compound (I) administration group - number of neutrophils of solvent administration group)/(number of neutrophils of LPS administration group - number of neutrophils of solvent administration group)} × 100

Table 1

| Administration group | Dose (mg/body) | Number of samples | Number of neutrophils* ($\times 10^5$ cells/BALF) | Inhibition ratio of increase in number of neutrophils |
|---|---|---|---|---|
| Solvent | - | 6 | $0.04 \pm 0.01$ | - |
| LPS | - | 5 | $1.31 \pm 0.19$ | - |
| Compound (1) | 0.1 | 6 | $0.72 \pm 0.22$ | 47% |

*: mean ± standard error

The number of neutrophils in the BALF of the LPS administration group was significantly increased compared with that of the solvent administration group. On the other hand, in Compound (I) administration group, the increase in number of neutrophils was lower than that of the LPS administration group. The increase in number of neutrophils by the LPS administration was inhibited by administering Compound (I). That is, it was shown that neutrophil infiltration of the lung can be inhibited by administering Compound (I).

It has been observed that the neutrophil infiltration of the lung, a tumor necrosis factor (TNF-α) which is a proinflammatory cytokine, and a macrophage inflammatory protein (MIP-2) which is a potent neutrophil chemotactic factor are increased by intratracheally administering the LPS [American Journal of Physiology (Am. J. Physiol.), 1999, vol. 276, p. L736]. These symptoms are the same as those in COPD patients [Trends in Pharmacological Sciences (Trends in Pharmacol. Sci.), 1998, vol. 19, p. 415]. Therefore, it is proposed that the LPS-induced pulmonary injury model is useful as an animal model of COPD.

In the BALF or the sputum of the COPD patient, many neutrophils are observed. When the patient has a greater number of neutrophils in the sputum or bronchial mucosa, airway obstruction worsens [American Review of Respiratory Diseases (Am. Rev. Respir. Dis.), 1989, vol. 140, p. 1527; American Journal of Respiratory and Critical Care Medicine (Am. J. Respir. Crit. Care Med.), 1996, vol. 153, p. 530; and American Journal of Respiratory and Critical Care Medicine (Am. J. Respir. Crit. Care Med.), 1998, vol. 158, p. 1277]. Furthermore, administration of elastase, which is released by neutrophils, to animals induces pulmonary emphysema-like symptoms [European Respiratory Journal (Eur. Respir. J.), 1985, vol. 132, p. 1155]. Consequently, it is suggested that inhibition of the neutrophil infiltration in the lung permits treatment of, for example, COPD, pulmonary emphysema, chronic bronchitis and the like.

In addition, since the LPS-induced pulmonary injury model exhibits neutrophilic inflammation, it is suggested that the LPS-induced pulmonary injury model is probably useful as an animal model of ARDS or ALI [Laboratory Animals (Lab. Anim.), 1992, vol. 26, p. 29; American Journal of Respiratory Cell and Molecular Biology (Am. J. Respir. Cell Mol. Biol.), 1997, vol. 16, p. 267; and Inflammation, 1999, vol. 23, p. 263].

TEST EXAMPLE 2: Inhibitory effect on increase of neutrophil in mainstream of cigarette smoke induced pulmonary injury model

Compound (I) was suspended to the concentration of 0.6 mg/mL in a physiological saline containing 25% Alevaire (registered trademark, Azwell, Osaka), which is an inhalant for respiratory organs, (administration solvent). The suspension was used for the test. The suspension is referred to as a "suspension for the administration of Compound (I)".

First, 0.5 mL of the administration solvent or the suspension for the administration of Compound (I) per kilogram of body weight was administered orally to male CD rats aged 6 to 7 weeks (Charles River Japan, Kanagawa). After 10 to 12 hours, the rats were systemically exposed to mainstream of cigarette smoke (Hi-Lite, Japan Tobacco

Inc., Tokyo) using a smoking exposure instrument (M.I.P.S, Osaka). The exposure to the mainstream of cigarette smoke for 5 minutes and then exposure to air for 10 minutes were successively performed by repeating them eight times.

A group subjected to the administration of the administration solvent and exposure to air instead of mainstream of cigarette smoke is referred to as a "solvent administration group". A group subjected to the administration of the administration solvent and exposure to mainstream of cigarette smoke is referred to as a "smoking group". A group subjected to the administration of the suspension for administration of Compound (I) and exposure to mainstream of cigarette smoke is referred to as a "Compound (I) administration group".

After the exposure for 6 hours, BALs were performed with Hanks' solution [Hanks' Balanced Salt Solution (Invitrogen Corporation, CA, USA)] (4 mL x 3 times). All of the BALFs were recovered and subjected to treatment as in Test example 1, and then the numbers of neutrophils were measured. The results are shown in Table 2. Similarly, cells in the BALFs of all individuals in this test were almost exclusively constituted of macrophages, neutrophils and lymphocytes. Eosinophils, basophils and other cells were hardly observed.

Table 2

| Administration group | Dose (mg/body) | Number of samples | Number of neutrophils* ($\times 10^5$ cells/BALF) | Inhibition ratio of increase in number of neutrophil |
|---|---|---|---|---|
| Solvent | - | 12 | $0.29 \pm 0.12$ | - |
| smoking | - | 12 | $0.56 \pm 0.23$ | - |
| Compound (I) | 0.3 | 12 | $0.34 \pm 0.13$ | 81% |

*: mean $\pm$ standard error

As a result of this test, the number of neutrophils in the BALF of the smoking group was significantly increased compared with that of the solvent administration group. In Compound (I) administration group, the increase in number of neutrophils observed in the smoking group was inhibited. That is, it was shown that neutrophil infiltration to the bronchoalveolar can be inhibited by administering Compound (I).

It is pointed out that COPD is caused by smoking [American Journal of Respiratory and Critical Care Medicine (Am. J. Respir. Crit. Care Med.), 2001, vol. 163, p. 1256]. It is also suggested that LPS in cigarette smoke is involved in the onset of COPD [Chest, 1999, vol. 115, p. 829]. It is proposed that the above-described pulmonary injury model in which neutrophil infiltration in the respiratory tract is caused by exposure of mainstream of cigarette smoke is useful in evaluating the therapeutic agent for COPD [Respiratory Research (Respir. Res.), 2001, vol. 2, p. E003; and Chest, 2002, vol. 121, p. 192S]. Therefore, it is proposed that Compound (I) is useful as an agent for the treatment and/or prevention of COPD.

As described above, Compound (I) or a pharmaceutically acceptable salt thereof is useful as a therapeutic and/or preventive agent for, for example, COPD, pulmonary emphysema, chronic bronchitis, ARDS, ALI and the like.

Compound (I) or a pharmaceutically acceptable salt thereof can be administered alone. However, usually, Compound (I) or a pharmaceutically acceptable salt thereof is preferably provided in various pharmaceutical preparations. Furthermore, these pharmaceutical preparations are used for animals and humans.

The pharmaceutical preparations according to the present invention may comprise Compound (I) or a pharmaceutically acceptable salt thereof alone as an active ingredient. Alternatively, the pharmaceutical preparations may comprise a mixture of Compound (I) or a pharmaceutically acceptable salt thereof with any effective ingredient used for another treatment. Furthermore, these pharmaceutical preparations are prepared by mixing the active ingredient (s) with one or more pharmaceutically acceptable carrier(s) and then employing any method well-known in the technical field of pharmaceutics.

As for administration routes, it is preferred to select the most effective route of administration. Examples of the administration routes include oral administration and parenteral administration such as intravenous, intratracheal, percutaneous administration and the like.

As for the dosage form, for example, tablets, injections, inhalants, external preparations and the like are included.

For example, the tablet suitable for oral administration can be prepared with, for example, excipients such as lactose and mannitol; disintegrants such as starch; lubricants such as magnesium stearate; binders such as hydroxypropylcellulose; surfactants such as a fatty ester; plasticizers such as glycerol; antiseptic agents such as benzoic acid and a phyroxybenzoate; and the like.

For example, the injection suitable for parenteral administration is preferably made of a sterilized aqueous solution containing the active compound, which is isotonic with the blood of the recipient. The solution used for the injection can be prepared with, for example, carriers such as a salt solution, a glucose solution or a mixture of a salt solution and a glucose solution.

The inhalant is prepared as follows: the active ingredient is prepared in the form of a powder or liquid, and mixed with a propellant for inhalation or a carrier. Then, the resulting mixture is charged into an appropriate inhaler such as a metered-dose inhaler or dry-powder inhaler. Also, when the active ingredient is in the form of a powder, a mechanical inhaler for powder may be generally used. When the active ingredient is in the form of a liquid, an inhaler such as a nebulizer may be used. A known propellant for inhalation may be widely used as the propellant for inhalation. Examples thereof include fron gases such as fron-11, fron-12, fron-21, fron-22, fron-113, fron-114, fron-123, fron-142c, fron-134a, fron-227, fron-C318 and 1,1,1,2-tetrafluoroethane; altanative fron gases such as HFA-227 and HFA-134a; hydrocarbon gases such as propane, isobutene and n-butane; diethyl ether; a nitrogen gas; a carbon dioxide gas and the like. A known carrier may be widely used as the carrier. Examples thereof include saccharides, sugar alcohols, amino acids and the like. Lactose, D-mannitol and the like are preferred.

Examples of the appropriate formulation of the external preparation include, but are not limited to, a cream form, a paste form, a jelly form, a gel form, an emulsion form, a liquid form and the like (ointment, liniment, lotion, etc.) that are prepared by dissolving or mixing the active ingredient with a base. Further examples of the formulation of the external preparation include a cataplasm, a tape formulation and the like, that are prepared by dissolving or mixing the active ingredient and a percutaneous-absorption promoter with a base and then applying the resulting mixture on a support composed of, for example, polyethylene, a polyester or a poly(ethylene terephthalate). Any base can be used as long as the base is pharmaceutically acceptable. A known base for the ointment, liniment, lotion or the like may be used as the above-described base. Examples thereof include sodium alginate; polymers such as gelatin, cornstarch, tragacanth gum, methylcellulose, hydroxyethylcellulose, carboxymethylcellulose, xanthan gum, dextrin, carboxymethyl starch, poly(vinyl alcohol), sodium polyacrylates, methoxyethylene-maleic anhydride copolymers, polyvinyl ethers and polyvinylpyrrolidones; fats and oils such as yellow beeswax, olive oils, cacao oils, sesame oils, soybean oil, camellia oils, peanut oils, beef tallow, lard and lanolin; Vaseline such as white vaseline and yellow vaseline; paraffin; hydrocarbon gel ointments (for example, trade mark: Plastibase, manufactured by Taisho Pharmaceutical Co., Ltd.); higher fatty acids such as stearic acid; higher alcohols such as cetyl alcohol and stearyl alcohol; polyethylene glycols; water and the like. As above-mentioned percutaneous-absorption promoter, any percutaneous-absorption promoter may be used so long as it is pharmaceutically acceptable. Examples thereof include alcohols such as methanol, ethanol, diethylene glycol and propylene glycol; polar solvents such as dimethyl sulfoxide and dodecylpyrrolidone; urea; esters such as ethyl laurate, isopropyl myristate and cetyl octanoate; Azone; olive oils and the like. Furthermore, if necessary, an inorganic filler such as kaolin, bentonite, zinc oxide and titanium oxide; a viscosity-adjusting agent; an antioxidant; a pH adjusting agent; a humectants such as a glycerol and propylene glycol, and the like may be added.

In these parenteral agents, at least one additive selected from the group consisting of a diluent, a flavor, an excipient, a disintegrant, a lubricant, a binder, a surfactant, a plasticizer, and an antiseptic as exemplarily described in the oral administration, may also be added.

The dosage and the frequency of dosage of Compound (I) or a pharmaceutically acceptable salt thereof may vary depending on, for example, the dosage form, the age and body weight of a patient, and the nature or severity of the symptom to be treated. Usually, in oral administration, Compound (I) or a pharmaceutically acceptable salt thereof is administered at a dose of 0.01 mg to 1 g and preferably 0.5 to 100 mg once a day or several times a day per an adult. In inhalation, Compound (I) or a pharmaceutically acceptable salt thereof is administered at a dose of 1 μg to 1,000 mg, preferably 0.01 to 100 mg, and more preferably 0.05 to 20 mg once a day or several times a day per an adult. In parenteral administration such as intravenous administration or the like, Compound (I) or a pharmaceutically acceptable salt thereof is administered at a dose of 1 μg to 100 mg and preferably 0.01 to 10 mg once a day or several times a day per an adult. However, the dosage and the frequency of dosage as described above vary depending on various conditions described above.

Aspects of the present invention will be described below based on Examples.

## Best Mode for Carrying Out the Invention

EXAMPLE 1: Tablet

According to a conventional method, tablets each having the following composition are prepared. First, 40 g of Compound (I), 286.8 g of lactose and 60 g of potato starch are mixed, and then 120 g of 10% aqueous solution of hydroxypropylcellulose is added thereto. The resulting mixture is kneaded, granulated and dried according to conventional method, followed by sized to form granules for tablet pressing. To the resulting granules are added 1.2 g of magnesium stearate and mixed. Tableting is performed with a tableting machine (model RT-15, manufactured by Kikusui Seisakusyo Ltd.) having a striker of 8 mm diameter to form tablets each containing 20 mg of active ingredient.

| | | |
|---|---|---|
| Prescription | Compound (I) | 20 mg |

(continued)

| | | |
|---|---|---|
| | Lactose<br>Potato starch<br>Hydroxypropylcellulose<br>Magnesium stearate | 143.4 mg<br>30 mg<br>6 mg<br>0.6 mg |
| | | 200 mg |

EXAMPLE 2: Injection

According to a conventional method, injections each having the following composition are prepared. First, 1 g of Compound (I) is dissolved in purified soybean oil, and then 12 g of purified egg-yolk lecithin and 25 g of glycerol for injection are added thereto. The volume of the mixture is adjusted to 1,000 mL by addition of distilled water for injection, and the resulting mixture is stirred and emulsified according to a conventional method. The resulting dispersion is aseptically filtrated through a disposable membrane filter having a pore size of 0.2 μm. Then, the resulting filtrate is aseptically filled into glass vials, each vial being filled with 2 mL of the filtrate. Consequently, injections each containing 2 mg of active ingredient per vial are obtained.

| | | |
|---|---|---|
| Prescription | Compound (I)<br>Purified soybean oil<br>Purified egg-yolk lecithin<br>Glycerol for injection<br>Distilled water for injection | 2 mg<br>200 mg<br>24 mg<br>50 mg<br>1.72 mL |
| | | 2.00 mL |

EXAMPLE 3: Dry-powder inhalant

First, 10 g of Compound (I) was pulverized using a JET MILL (model A-0 JET, manufactured by Seishin Enterprise Co., Ltd.) at an air pressure of 5 $kg/cm^2$ and a feed speed of 1.5 g/min (volume average particle size: 5.7 μm). The resulting pulverized Compound (I) and lactose (Pharmatose 325M: registered trademark, manufactured by DMV) are mixed in weight ratio of 1 to 5 to form a dry-powder formulation. The formulation may be administered by a common dry-powder inhaler.

| | | |
|---|---|---|
| Prescription | Compound (I)<br>Lactose | 16.7 mg<br>83.3 mg |
| | | 100 mg |

Industrial Applicability

The present invention provides a therapeutic and/or preventive agent for pulmonary disease that exhibits neutrophilic inflammation, such as, COPD, pulmonary emphysema, chronic bronchitis, ARDS, ALI and the like, which comprises 7-[2-(3,5-dichloro-4-pyridyl)-1-oxoethyl]-4-methoxy-spiro[1,3-benzodioxol-2,1'-cyclopentane] or a pharmaceutically acceptable salt thereof as an active ingredient.

## Claims

**1.** A therapeutic and/or preventive agent for pulmonary disease that exhibits neutrophilic inflammation, which comprises 7-[2-(3,5-dichloro-4-pyridyl)-1-oxoethyl]-4-methoxy-spiro[1,3-benzodioxol-2,1'-cyclopentane] represented by formula (I)

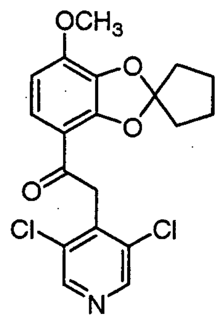

(I)

or a pharmaceutically acceptable salt thereof as an active ingredient.

2. The therapeutic and/or preventive agent for pulmonary disease that exhibits neutrophilic inflammation according to Claim 1, wherein the pulmonary disease that exhibits neutrophilic inflammation is a disease selected from the group consisting of chronic obstructive pulmonary disease (COPD), pulmonary emphysema and chronic bronchitis.

3. The therapeutic and/or preventive agent for pulmonary disease that exhibits neutrophilic inflammation according to Claim 1, wherein the pulmonary disease that exhibits neutrophilic inflammation is acute respiratory distress syndrome (ARDS) or acute lung injury (ALI).

4. A method for treating and/or preventing pulmonary disease that exhibits neutrophilic inflammation, which comprises administering an effective amount of 7-[2-(3,5-dichloro-4-pyridyl)-1-oxoethyl]-4-methoxy-spiro[1,3-benzodioxol-2,1'-cyclopentane] represented by formula (I)

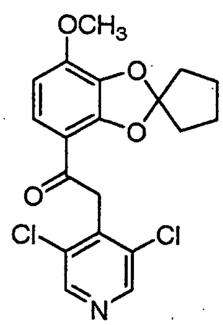

(I)

or a pharmaceutically acceptable salt thereof.

5. The method for treating and/or preventing pulmonary disease according to Claim 4, wherein the pulmonary disease that exhibits neutrophilic inflammation is a disease selected from the group consisting of COPD, pulmonary emphysema and chronic bronchitis.

6. The method for treating and/or preventing pulmonary disease according to Claim 4, wherein the pulmonary disease that exhibits neutrophilic inflammation is ARDS or ALI.

7. Use of 7-[2-(3,5-dichloro-4-pyridyl)-1-oxoethyl]-4-methoxy-spiro[1,3-benzodioxol-2,1'-cyclopentane] represented by formula (I)

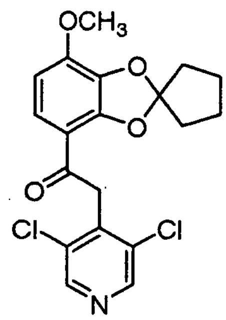

( I )

or a pharmaceutically acceptable salt thereof, for the manufacture of a therapeutic and/or preventive agent for pulmonary disease that exhibits neutrophilic inflammation.

**8.** Use of 7-[2-(3,5-dichloro-4-pyridyl)-1-oxoethyl]-4-methoxy-spiro[1,3-benzodioxol-2,1'-cyclopentane] or a pharmaceutically acceptable salt thereof according to Claim 7, wherein the pulmonary disease that exhibits neutrophilic inflammation is a disease selected from the group consisting of COPD, pulmonary emphysema and chronic bronchitis.

**9.** Use of 7-[2-(3,5-dichloro-4-pyridyl)-1-oxoethyl]-4-methoxy-spiro[1,3-benzodioxol-2,1'-cyclopentane] or a pharmaceutically acceptable salt thereof according to Claim 7, wherein the pulmonary disease that exhibits neutrophilic inflammation is ARDS or ALI.

## INTERNATIONAL SEARCH REPORT

International application No.
PCT/JP2004/004611

A. CLASSIFICATION OF SUBJECT MATTER
Int.Cl$^7$ A61K31/443, A61P11/00//C07D405/06

According to International Patent Classification (IPC) or to both national classification and IPC

B. FIELDS SEARCHED

Minimum documentation searched (classification system followed by classification symbols)
Int.Cl$^7$ A61K31/00-31/80, 45/00-45/08, A61P1/00-43/00, C07D405/00-405/14

Documentation searched other than minimum documentation to the extent that such documents are included in the fields searched

Electronic data base consulted during the international search (name of data base and, where practicable, search terms used)
MEDLINE(STN), EMBASE(STN), BIOSIS(STN), BIOTECHABS(STN), CAplus(STN), REGISTRY(STN), WPI(DIALOG), JSTPLUS(JOIS), JMEDPLUS(JOIS)

C. DOCUMENTS CONSIDERED TO BE RELEVANT

| Category* | Citation of document, with indication, where appropriate, of the relevant passages | Relevant to claim No. |
|---|---|---|
| Y | WO 96/36624 A1 (Kyowa Hakko Kogyo Co., Ltd.), 21 November, 1996 (21.11.96), Example 140; description, page 1, line 4 to page 3, line 23 | 1-3,7-9 |
| P,Y | WO 03/066044 A1 (BOERINGER INGELHEIM PHARM GMBH. & CO. KG.), 14 August, 2003 (14.08.03), Example 140; description, page 26, line 26 to page 27, line 2 | 1-3,7-9 |
| P,Y | WO 2004/005276 A1 (Kyowa Hakko Kogyo Co., Ltd.), 15 January, 2004 (15.01.04) | 1-3,7-9 |

[X] Further documents are listed in the continuation of Box C.   [X] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 June, 2004 (11.06.04) | 06 July, 2004 (06.07.04) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No. PCT/JP2004/004611

C (Continuation). DOCUMENTS CONSIDERED TO BE RELEVANT

| Category* | Citation of document, with indication, where appropriate, of the relevant passages | Relevant to claim No. |
|---|---|---|
| Y | WO 03/016279 A1 (Tanabe Seiyaku Co., Ltd.), 27 February, 2003 (27.02.03), Claims; examples | 1-3,7-9 |
| Y | WO 02/098880 A1 (BAYER AG.), 12 December, 2002 (12.12.02), Claims; description, page 1, line 7 to page 2, line 5 | 1-3,7-9 |
| Y | JP 2002-537383 A (Merck Frosst Canada and Co.), 05 November, 2002 (05.11.02), Claims | 1-3,7-9 |
| Y | WO 00/51598 A1 (SMITHKLINE BEECHAM CORP.), 08 September, 2000 (08.09.00), Full text | 1,2,7,8 |
| Y | Masayoshi ISHIBASHI et al., "Mansei Heisokusei Haishikkan no Chiryo Theophylline to Atarashii Phosphodiesterase IV Sogaiyaku", Gendai Iryo, 2002, 34(9), pages 2249 to 2254; particularly, pages 2253 to 2254 | 1,2,7,8 |
| Y | Naomi YAMASHITA, "Tiotropium to Phosphodiesterase IV Sogaiyaku", Biomedicine & Therapeutics, 2001, 35(11), pages 1225-1226; full text | 1,2,7,8 |
| Y | Yukio NAGASAKA, "Kokyuki Shikkan ni Taisuru Theophylline no Koka", Kokyu to Junkan, 1996, 44(8), pages 839 to 845; full text | 1,2,7,8 |
| P,Y | WO 03/080049 A1 (CELGENE CORP.), 02 October, 2003 (02.10.03), Claims; description, page 12, line 12 to page 18, line 24 | 1,2,7,8 |
| Y | HAEFNER, D. et al., Additive Effects of Phosphodiesterase-4 Inhibition on Effects of rSP-C Surfactant. Am. J. Respir. Crit. Care Med., 2000, 161, pages 1495 to 1500; particularly, summary | 1,3,7,9 |
| Y | TEIXEIRA, M.M. et al., Phopshodiesterase(PDE)4 inhibitors: anti-inflammatory drugs of the future? TiPS, 1997, (18), pages 164 to 170; full text | 1,3,7,9 |

Form PCT/ISA/210 (continuation of second sheet) (January 2004)

**INTERNATIONAL SEARCH REPORT**

International application No.
PCT/JP2004/004611

**Box No. II Observations where certain claims were found unsearchable (Continuation of item 2 of first sheet)**

This international search report has not been established in respect of certain claims under Article 17(2)(a) for the following reasons:

1. [X] Claims Nos.: 4-6
because they relate to subject matter not required to be searched by this Authority, namely:
The inventions as set forth in claim 4-6 pertain to methods for treatment of the human body by therapy. (Article 17 (2)(a)(i) of the PCT and Rule 39.1 (iv) of the Regulations under the PCT)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (2)) (January 2004)

**INTERNATIONAL SEARCH REPORT**
Information on patent family members

International application No.
PCT/JP2004/004611

| | | |
|---|---|---|
| WO 96/36624 A1 | 1996.11.21 | AU 9657029 A<br>NO 9700151 A<br>EP 771794 A1<br>KR 97704724 A<br>AU 705690 B<br>US 2002/0128290 A1<br>US 6514996 B2<br>CN 1154697 A<br>US 6716987 B1 |
| WO 03/066044 A1 | 2003.08.14 | DE 10205274 A1<br>US 2003/0203918 A1<br>AU 2003205717 A1 |
| WO 2004/005276 A1 | 2004.01.15 | (Family: none) |
| WO 03/016279 A1 | 2003.02.27 | JP 2003-119180 A<br>JP 2003-119195 A<br>JP 2003-119196 A<br>EP 1424326 A1 |
| WO 02/098880 A1 | 2002.12.12 | EP 1397363 A1 |
| JP 2002-537383 A | 2002.11.05 | WO 00/50402 A1<br>AU 200027891 A<br>US 6204275 B1<br>EP 1157007 A1<br>AU 764005 B |
| WO 00/51598 A1 | 2000.09.08 | AU 200033869 A<br>EP 1156799 A1<br>NO 200104222 A<br>SK 200101237 A3<br>BR 200008603 A<br>CZ 2001031449 A3<br>KR 2001102459 A<br>HU 200200288 A2<br>CN 1349403 A<br>JP 2002-538114 A<br>ZA 200107186 A<br>MX 2001008853 A1<br>US 6670394 B1<br>NZ 513695 A |
| WO 03/080049 A1 | 2003.10.02 | US 2003/0187052 A1<br>AU 2003224729 A1 |

Form PCT/ISA/210 (patent family annex) (January 2004)